# EUROPEAN PATENT APPLICATION

(11) **EP 1 405 854 A1**
(43) Date of publication of application: **07.04.2004**
(21) Application number: 02733309.5
(22) Date of filing: 05.06.2002
(51) Int. Cl.: C07F 9/50, B01J 31/22, C07C 45/50, C07C 47/21

(54) **LITHIUM P-DIARYLPHOSPHINOBENZENESULFONATES, PROCESS FOR PREPARATION OF THE SAME AND USE THEREOF**

(30) Priority: 07.06.2001 JP 2001172229; 28.08.2001 JP 2001257197
(71) Applicant: Kuraray Co., Ltd., Kurashiki-City, Okayama 710-8622 (JP)
(72) Inventor: TSUJI, Tomoaki, Kurashiki-shi, Okayama 710-0801 (JP); MATSUMOTO, Masayuki, Kurashiki-shi, Okayama 710-0801 (JP); SUZUKI, Shigeaki, c/o Kuraray Co., Ltd., Kurashiki-shi, Okayama 710-0801 (JP)
(74) Representative: VOSSIUS & PARTNER
(86) International application number: PCT/JP2002/005543
(87) International publication number: WO 2002/100872

(57) **Abstract**

Lithium p-diarylphosphinobenzenesulfonates represented by the general formula (I) 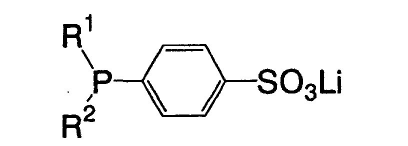 wherein R¹ and R² are each optionally substituted aryl; a process for preparing the same; a group VIII metal complex comprising a group VIII metal compound and the above lithium p-diarylphosphinobenzenesulfonate coordinating thereto and a process for hydroformylation with the complex. Hydroformylation with the complex permits easy and industrially advantageous production of aldehydes.

## Description

### TECHNICAL FIELD

The present invention relates to lithium p-diarylphosphinobenzenesulfonates, processes for preparation of the same and use thereof.

The lithium p-diarylphosphinobenzenesulfonates provided by the present invention are useful as ligands for hydroformylation catalysts. Accordingly, the above uses include hydroformylation catalysts comprising the lithium p-diarylphosphinobenzenesulfonates provided by the present invention as the catalyst component, and process for producing aldehydes by using such hydroformylation catalysts. On performing hydroformylation of 7-octen-1-al with use of a rhodium complex having as ligand a lithium p-diarylphosphinobenzenesulfonate, one can obtain a linear aldehyde product of 1,9-nonanedial with good selectivity. Since lithium p-diarylphosphinobenzenesulfonates have high solubility in water, they can readily be recovered by water extraction from the reaction mixture of the above hydroformylation and re-used.

### BACKGROUND ART

Reaction of ethylenically unsaturated compounds with hydrogen and carbon monoxide in the presence of a catalyst comprising a group VIII metal compound with or without modification by a ligand such as organic phosphorus compound, to obtain aldehydes, is known as hydroformylation or oxo reaction. Production of aldedhydes by this reaction has been of high commercial value.

Since group VIII metal compounds are very expensive, their use as catalysts for commercial hydroformylation requires that they be recovered after use at high yields and re-used. Hydroformylation catalysts have therefore been recovered by an evaporation separation process which comprises distilling off the product and unreacted starting materials from the reaction mixture and recovering the hydroformylation catalysts as evaporation residue.

Hydroformylation catalysts are, however, unstable against heat and suffer a significant decrease in activity due to heat when the desired products have high boiling point. It is generally said that the evaporation separation process is applicable only to hydroformylation of ethylenically unsaturated compounds having not more than 5 carbon atoms.

Use of catalysts comprising group VIII metal compounds modified by a water-soluble ligand has then attracted attention in commercial processes of hydroformylation of ethylenically unsaturated compounds having at least 6 carbon atoms. In order for this process to achieve commercial value, it is however necessary to overcome two opposing principles: increasing the reaction rate requires that the water-soluble catalyst and lipophilic starting materials be present in the same phase; and on catalyst recovery, the water-soluble catalyst and the lipophilic product must be in separate phases. Various processes have been proposed for this purpose.

For example, (1) Japanese Patent No. 2857055 describes a process which comprises the steps of hydroformylating 7-octen-1-al in the presence of a rhodium compound, a phosphorus ligand having a sulfonic acid group and polyalkylene glycol derivatives, adding water to the reaction mixture and separating the catalyst components by extraction and removing water from the aqueous layer thus separated to obtain the polyalkylene glycol derivative containing the catalyst component and circulating this to the reactor for re-use and, at the same time, obtaining 1,9-nonanedial from the organic layer. (2) WO94/17081 and Japanese Patent TOKUHYOU 506110/1996 disclose a hydroformylation process which comprises using a group VIII noble metal-ligand complex catalyst which is a complex with a ligand of potassium p-diphenylphosphinobenzenesulfonate.

The above process (1) can, according to Example of the patent, perform hydroformylation in a low rhodium concentration of not more than 25 ppm with use of sodium m-diphenylphosphinobenzenesulfonate as a water-soluble ligand and recover and re-use the catalyst. The resultant reaction mixture contains, besides unreacted starting material and the reaction product, the polyalkylene glycol derivatives used in an amount of only 10% of the resultant reaction mixture. The process therefore has high volume efficiency. However, this process has the problem that the ratio of the desired linear aldehyde to branched aldehydes remains to be 3.5 at most.

The above process (2) carries out, according to its Example, hydroformylation in a heterogeneous system with use of potassium p-diphenylphosphinobenzenesulfonate as a water-soluble ligand and in the presence of an aqueous sodium hydrogencarbonate solution having dissolved lauric acid, and a cosolvent of isopropyl alcohol. This process has an advantage of high ratio of the linear aldehyde to branched aldehydes of about 13. However, with a high rhodium concentration in the aqueous layer of 500 ppm and with presence of about 0.5 part by weight of the water layer based on one part by weight of the starting material olefin, this process suffers a poor volume efficiency. There is no description with respect to the efficiency of catalyst recovery. According to the knowledge possessed by the present inventors, use of potassium p-diphenylphosphinobenzenesulfonate, which has a low solubility in water, in the above process (1) requires a large amount of water to recover by extraction the salt from the reaction mixture after the hydroformylation has completed and like complex recovery processes and, besides, results in a low recovery ratio.

It has therefore been desired to develop a process to solve the above problems, that is, a process which can perform reaction at a low rhodium concentration and have high volume efficiency and good catalyst recovery efficiency, thus being excellent in economy, to obtain the desired linear aldehyde with high selectivity.

Accordingly, an object of the present invention is to provide a novel ligand for hydroformylation catalysts that have, in hydroformylation of ethylenically unsaturated compounds, excellent economy and giving the desired linear aldehydes with high selectivity.

Another object of the present invention is to provide a process for producing the above ligand with high purity, commercially advantageously at high yield.

Still another object of the present invention is to provide a group VIII metal complex to be used for hydroformylation of ethylenically unsaturated compounds, said complex having excellent economy and giving the desired linear aldehydes at high selectivity.

Yet another object of the present invention is to provide a process for producing aldehydes readily and commercially advantageously by the hydroformylation utilizing the above group VIII metal complex.

A further object of the present invention is to provide a process for recovering at high yield the catalyst components used for the hydroformylation from the reaction mixture.

### DISCLOSURE OF THE INVENTION

The present invention provides a lithium p-diarylphosphinobenzenesulfonate represented by the general formula (I) (hereinafter sometimes referred to as "lithium sulfonate (I)" 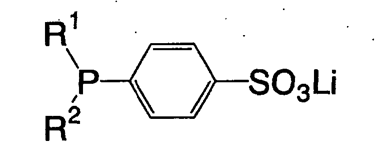 wherein R¹ and R² each represents an aryl group which may be substituted.

The present invention also provides a process for producing lithium sulfonates (I) which comprises reacting a potassium p-diarylphosphinobenzenesulfonate represented by the general formula (II) (hereinafter sometimes referred to as "potassium sulfonate (II)) 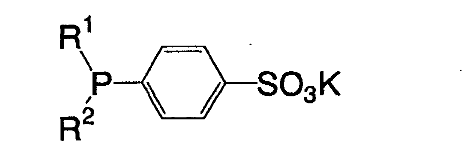 wherein R¹ and R² are as defined above,
with the lithium salt of an acid having a solubility in water of 5 to 30% by weight, in the presence of water or a mixture of water and a water-miscible organic solvent.

The present invention further provides a group VIII metal complex comprising a group VIII metal compound and, coordinating thereto, a lithium sulfonate (I)(hereinafter sometimes referred to as "group VIII metal complex (I)").

The present invention still further provides a process for producing aldehydes which comprises, on hydroformylating ethylenically unsaturated compounds with carbon monoxide and hydrogen in the presence of a catalyst to produce the corresponding aldehydes, using the group VIII metal complex (I) as the catalyst.

The present invention yet further provides a process for recovering the above catalyst components which comprises, on recovering catalyst components from the reaction mixture obtained by the above process for producing aldehydes, contacting the reaction mixture with water to extract the catalyst components into an aqueous layer and then removing the water from the aqueous layer.

### MODES FOR CARRYING OUT THE INVENTION

Examples of the aryl groups represented by R¹ and R² are phenyl, naphthyl and anthryl, of which phenyl is most preferable. These aryl groups may be substituted. Examples of the substituents are halogen atoms, e.g. fluorine, chlorine, bromine and iodine; alkyl groups, e.g. methyl, ethyl, propyl, isopropyl, butyl, isobutyl, s-butyl, t-butyl and cyclohexyl; fluoroalkyl groups, e.g. difluoromethyl, trifluoromethyl, 1,1-difluoroethyl, 2,2-difluoroethyl and 1-fluoropropyl; alkoxy groups, e.g. methoxy, ethoxy, propoxy, isopropoxy, butoxy, isobutoxy, s-butoxy and t-butoxy; acyl groups, e.g. acetyl, propionyl, butyryl, isobutyryl and pivaloyl; acyloxy groups, e.g. acetyloxy, propionyloxy, butyryloxy and isobutyryloxy; alkoxycarbonyl groups, e.g. methoxycarbonyl, ethoxycarbonyl, propoxycarbonyl, isopropoxycarbonyl, butoxycarbonyl, isobutoxycarbonyl, s-butoxycarbonyl and t-butoxycarbonyl; amino groups, e.g. amino, methylamino, dimethylamino and acetylamino; carboxylic groups and metal salts thereof; sulfonic acid groups and metal salts thereof; and phosphorous acid groups and metal salts thereof.

Examples of the lithium sulfonates (I) are lithium p-diphenylphosphinobenzenesulfonate, lithium p-bis(o-tolyl)phosphinobenzenesulfonate, lithium p-bis(m-tolyl)phosphinobenzenesulfonate, lithium p-bis(p-tolyl)phosphinobenzenesulfonate, lithium p-bis(p-methoxyphenyl)phosphinobenzenesulfonate, lithium p-bis(p-fluorophenyl)phosphinobenzenesulfonate, lithium p-bis(p-trifluoromethylphenyl)phosphinobenzenesulfonate, dilithium bis(p-sulfophenyl)phenylphosphide and dilithium bis(p-sulfophenyl)(p-trifluoromethylphenyl)phosphide. Among these lithium sulfonates (I), lithium p-diphenylphosphinobenzenesulfonate is particularly preferred.

The process for producing the lithium sulfonates (I) is now described.

It is necessary to use, as the lithium salt of an acid, those having a solubility in water at 20°C of 5 to 30% by weight based on 100 g of water. Where the lithium salt of an acid that can form hydrate is used, the above solubility means that the solubility of the anhydride is in a range of 5 to 30% by weight based on 100 g of water. Use of such lithium salt of an acid renders it possible, after completion of reaction of a potassium sulfonate (II) and the lithium salt of the acid, to separate by filtration the starting material lithium salt of the acid and byproduced potassium salt of the acid from the reaction mixture, and hence to isolate the desired lithium sulfonate (I) readily.

Examples of the lithium salt of an acid are carbonate, phosphate, phosphite, diphosphate, sulfate, sulfite and hydrochloride. Of these, carbonate, sulfate and sulfite are preferred. The lithium salt of an acid is used preferably in such an amount as to insure that the number of lithium atoms in the lithium salt falls within a range of 4 to 40, more preferably 6 to 10, based on 1 atom of the potassium atom present in the potassium sulfonate (II) used, which amount leads to better operability and yield.

This reaction is carried out in the presence of water or a mixture of water and a water-miscible organic solvent. Examples of usable water-miscible organic solvents are alcohols, e.g. methyl alcohol, ethyl alcohol, propyl alcohol, ethylene glycol and diethylene glycol; ketones, e.g. acetone, ethyl methyl ketone and dimethyl ketone; esters, e.g. methyl formate, ethyl formate, methyl acetate, ethyl acetate, methyl propionate and ethyl propionate; and ethers, e.g. diethyl ether, ethylene glycol monomethyl ether, ethylene glycol dimethyl ether, ethylene glycol monoethyl ether, ethylene glycol diethyl ether, ethylene glycol ethyl methyl ether diethylene glycol dimethyl ether, diethylene glycol ethyl methyl ether and diethylene glycol diethyl ether. These organic solvents are preferably poor solvents for the lithium salt of an acid used. These organic solvents may be used singly or in combination of 2 or more. Water is used preferably in an amount of 1 to 50 parts by weight based on 1 part of the potassium sulfonate (II) used, more preferably in an amount of 2 to 20 parts by weight on the same basis. The organic solvent is used preferably in an amount of 1 to 50 parts by weight based on 1 part of the potassium sulfonate (II) used, more preferably in an amount of 2 to 20 parts by weight on the same basis. The ratio of the amount of the organic solvent to that of water is preferably not more than 3 parts by volume, more preferably not more than 1.5 parts by volume.

The reaction temperature can be appropriately selected within a range up to the reflux temperature of the solvent used. However, the reaction is carried out preferably at the reflux temperature in view of reaction efficiency. The reaction time is preferably in a range of 0.5 to 10 hours, more preferably in a range of 1 to 4 hours.

Although the reaction may be carried out under the atmosphere, it is desirably performed under an atmosphere of an inert gas such as nitrogen or argon, which suppresses oxidation of the lithium sulfonate (I) used.

Thus, the reaction is preferably performed under an atmosphere of inert gas such as nitrogen or argon and in the presence of water or a mixture of water and a water-miscible organic solvent, by mixing a potassium sulfonate (I) and the lithium salt of an acid and then stirring the resulting mixture at a prescribed temperature.

The lithium sulfonate (I) thus obtained can be isolated and purified by any usual process employed in isolation and purification of organic compounds. For example, the product can be isolated by the successive steps of condensing the reaction mixture, adding a poor solvent for metal salts of acids, such as methyl alcohol, ethyl alcohol, propyl alcohol or acetone, separating solid matter by filtration and condensing the filtrate.

The lithium sulfonate (I) obtained by the above process, which has high purity and low chlorine content, can be used as it is as a ligand for noble metal catalysts.

The starting material potassium sulfonate (II) can be produced by any known process, for example, (1) by a process which comprises reacting a chlorodiarylphosphine with metallic sodium to obtain a sodium diarylphosphide, reacting the sodium diarylphosphide with lithium p-chlorobenzenesulfonate in the presence or absence of the potassium salt of an acid, and reacting the obtained reaction mixture by adding an aqueous solution of the potassium salt of an acid or a mixture of the potassium salt of an acid and water; and (2) by a process which comprises reacting a chlorodiarylphosphine with metallic potassium to obtain the corresponding potassium diarylphosphide and then reacting the potassium diarylphosphide with lithium p-chlorobenzenesulfonate (see WO94/17081 and Japanese Patent TOKUHYOU 506110/1996).

The group VIII metal complex (I) comprising a group VIII metal compound and, coordinating thereto, the lithium sulfonate (I) has the catalytic activity to accelerate hydroformylation of ethylenically unsaturated compounds. The group VIII metal complex (I) is water-soluble and can be used for known hydroformylation utilizing a catalyst comprising a group VIII metal compound modified with a water-soluble ligand.

The group VIII metal compound used for this purpose should either originally have the catalytic activity to accelerate hydroformylation of ethylenically unsaturated compounds or acquire such catalytic activity under reaction conditions for the hydroformylation. Examples of such metal compound are those rhodium compounds, cobalt compounds, ruthenium compounds and iron compounds that have been used as catalysts for hydroformylation. Examples of the rhodium compounds are rhodium oxides, e.g. RhO, Rh₂O, Rh₂O₃ and RhO₂; rhodium salts, e.g. rhodium nitrate, rhodium sulfate, rhodium chloride, rhodium iodide and rhodium acetate; and rhodium complexes, e.g. Rh₄(CO)₁₂, Rh₆(CO)₁₆, RhCl(CO)(PPh₃)₂, RhCl(PPh₃)₃, RhBr(CO)(PPh₃)₂, RhCl(CO)(AsPPh₃)₂ and Rh(acac)(CO)₂. Examples of the cobalt compounds are cobalt complexes, e.g. HCo(CO)₄, Co₂(CO)₈, HCo(CO)₃ and HCo₃(CO)₉. Examples of the ruthenium compounds are ruthenium complexes, e.g. Ru₃(CO)₁₂, Ru(CO)₃(PPh₃)₂, RhCl₃(PPh₃)₃ and RuCl₂(PPh₃)₃. Examples of the iron compounds are iron complexes, e.g. Fe(CO)₅, Fe(CO)₄PPh₃ and Fe(CO)₄(PPhs)₂. Among these compounds, it is preferable to use rhodium compounds, for which mild conditions are sufficient for hydroformylation, in particular Rh(acac)(CO)₂.

The above lithium sulfonates (I) may be used singly or in combination of 2 or more, or further in combination with a phosphine, e. g. triisopropylphosphine, tributylphosphine, tri-t-butylphosphine, tribenzylphosphine, triphenylphosphine, tris(p-methoxyphenyl)phosphine, tris(p-N,N-dimethylaminophenyl)phosphine, tris(p-fluorophenyl)phosphine, tris(p-chlorophenyl)phosphine, tri-o-tolylphosphine, tri-m-tolylphosphine, tri-p-tolylphosphine, tris(pentafluorophenyl)phosphine, bis(pentafluorophenyl)phenylphosphine, diphenyl(pentafluorophenyl)phosphine, methyldiphenylphosphine, ethyldiphenylphosphine, cyclohexyldiphenylphosphine, dimethylphenylphosphine, diethylphenylphosphine, 2-furyldiphenylphosphine, 2-pyridyldiphenylphosphine, 4-pyridyldiphenylphosphine, m-diphenylphosphinobenzenesulfonic acid or metal salts thereof, p-diphenylphosphinobenzoic acid or metal salts thereof and p-diphenylphosphinophenylphosphoric acid or metal salts thereof; or a phosphite, e.g. triethyl phosphite, triphenyl phosphite, tris(p-methoxyphenyl) phosphite, tris(p-methylphenyl) phosphite, tris(p-trifluoromethylphenyl) phosphite, tris(2,4-dimethylphenyl) phosphite and tris(2,4-di-t-butylphenyl) phosphite.

The lithium sulfonate (I) is used preferably in an amount of 1 to 10000 moles in terms of phosphorus atom per mole of the group VIII metal compound used in terms of said group VIII metal atom, more preferably 2 to 1000 moles in the same terms. If the amount of the lithium sulfonate (I) is less than this range, the stability of the catalyst will be impaired. If the amount exceeds this range, the reaction rate will tend to decrease.

There are no specific restrictions with respect to the preparation process for the group VIII metal complex (I). For example, the complex can be prepared by a process which comprises separately preparing a solution of a group VIII metal compound in a solvent that does not influence the hydroformylation and a solution of a lithium sulfonate (I) in the same solvent, introducing the two solutions into a hydroformylation reactor and effecting reaction therein to obtain a complex. The complex can also be prepared by introducing a lithium sulfonate (I) into the above group VIII metal compound solution and then adding a solvent that does not affect the hydroformylation, to obtain a homogeneous solution.

The process for producing the aldehydes is described next.

Ethylenically unsaturated compounds usable for this process can be any of linear, branched and cyclic terminal olefins or inner olefins. Examples of such ethylenically unsaturated compounds are unsaturated aliphatic hydrocarbons, e.g. ethylene, propylene, 1-butene, 1-pentene, 1-hexene, 1-heptene, 1-octene, 1-nonene, 2-butene, isobutene, 2-octene, 1,7-octadiene, vinylcyclohexene, cyclooctadiene, dicyclopentadiene, butadiene polymers and isoprene polymers; styrenes, e.g. styrene, α-methylstyrene, β-methylstyrene, alkyl group-ring substituted styrenes and divinylbenzene; alicyclic olefin hydrocarbons, e.g. cyclopentene, cyclohexene, 1-methylcyclohexene, cyclooctene and limonene; and functional group-containing olefins, e.g. allyl alcohol, crotyl alcohol, 3-methyl-3-buten-1-ol, 7-octen-1-ol, 2,7-octadienol, vinyl acetate, allyl acetate, methyl acrylate, ethyl acrylate, methyl methacrylate, allyl acrylate, vinyl methyl ether, allyl ethyl ether, 5-hexenamide, acrylonitrile and 7-octen-1-al.

With respect to the H₂/CO molar ratio of the mixed gas of hydrogen and carbon monoxide used for the hydroformylation, it is desirable that the ratio in the inlet gas be in a range of 0.1 to 10, more preferably 0.5 to 2, which permits easy maintenance of the mixed gas composition. The reaction pressure is desirably in a range of 0.1 to 10 MPa, more preferably 0.5 to 5 MPa, in view of reaction rate. The reaction temperature is desirably in a range of 40 to 150°C, more preferably 60 to 130°C, which suppresses deactivation of the catalyst. The reaction can be carried out in a stirred-type, liquid circulation-type, gas circulation-type, bubbled column-type or like reactors. The reaction can be carried out either continuously or batch-wise.

The group VIII metal complex (I) is used desirably in an amount of 0.0001 to 1000 milligram-atom in terms of group VIII metal atom per liter of the reaction liquid, more preferably in an amount of 0.005 to 10 milligram-atom in the same terms. Too small an amount results in too low a reaction rate, while the amount exceeding this range, which increases the catalyst cost, no longer increases the reaction rate efficiently.

It is desirable, on hydroformylation of lipophilic ethylenically unsaturated compounds with use of the group VIII metal complex (I) of the present invention, to permit a solvent to be present in the reaction zone. Examples of solvents usable for this purpose are aprotic polar solvents, e.g dimethyl sulfoxide, N-methylpyrrolidone, sulfolane, dimethylformamide, acetonitrile, acetone, dioxane and tetrahydrofuran; alcohols, e.g. methyl alcohol, ethyl alcohol, propyl alcohol, isopropyl alcohol, butyl alcohol, s-butyl alcohol and t-butyl alcohol; glycols, e.g. ethylene glycol, propylene glycol, diethylene glycol, ethylene glycol monomethyl ether, ethylene glycol dimethyl ether, diethylene glycol diethyl ether, triethylene glycol, triethylene glycol dimethyl ether, tetraethylene glycol and tetraethylene glycol dimethyl ether; and polyalkylene glycols, e.g. polyethylene glycol, polypropylene glycol, polyethylene glycol monomethyl ether and polyethylene glycol dimethyl ether. These solvents may be used singly or in combination or 2 or more. Among these solvents, it is desirable to use polyalkylene glycols, such as polyethylene glycol and polyethylene glycol dimethyl ether, which usage prevents precipitation of catalyst components and increases the efficiency of catalyst recovery. These solvents are used desirably in an amount of 2 to 50% by volume, more preferably 5 to 20% by volume, in the mixed reaction liquid for the hydroformylation.

Although there are no specific restrictions with respect to the method of feeding starting materials, it is desirable to feed an ethylenically unsaturated compound, a group VIII metal complex (I) solution prepared separately and, as necessary, a solvent and then introduce a mixed gas of hydrogen and carbon monoxide under a prescribed pressure. Then the reaction is desirably effected with stirring at a prescribed temperature in a homogeneous system.

The aldehydes obtained by the above process can be isolated and purified by any of the usual processes employed for isolation and purification of organic compounds. For example, the product is isolated and purified from the organic layer that has been obtained via the recovery process for the catalyst components to be described next, by distillation, recrystallization, column chromatography or like processes.

The process for recovering the catalyst components is described next.

In the present invention, the term 'catalyst components' means the group VIII metal complex (I) and the lithium sulfonate (I) which is usually used in excess against the group VIII metal compound.

On recovery of catalyst components from the reaction mixture of the hydroformylation, at first water is added to the reaction mixture after the hydroformylation. The amount of the water added is, though not particularly restricted, desirably in a range of 1 to 200% by volume, more preferably in a range of 5 to 50% by volume, based on the volume of the reaction mixture, in consideration of operability and the solubility of the catalyst components in water.

The reaction mixture is then contacted with the water with stirring or by like methods to extract the catalyst components with water. On this occasion, the extraction is desirably effected at a temperature of 20 to 90°C and under an atmosphere of an inert gas such as nitrogen, helium or argon or a mixed gas comprising hydrogen and carbon monoxide.

Then, an organic layer containing the reaction product of the hydroformylation and an aqueous layer containing the catalyst components are permitted to separate from each other. If, during the extraction procedure, the organic layer and the aqueous layer will not sufficiently separate from each other by being kept standing, the separation may be accelerated by centrifuge or like means. The separation can also be accelerated by adding a hydrocarbon having a small specific gravity compared to water such as hexane or cyclohexane.

The organic layer contains, other than the reaction product, unreacted ethylenically unsaturated compound and small amounts of the catalyst components. It is therefore desirable, in order to increase the recovery ratio of the catalysts components, to wash the organic layer with water and add the washings to the aqueous layer.

Removal of water from the aqueous layer thus obtained achieves recovery of the catalyst components. The removal of water is carried out by the usual process such as distillation under reduced pressure. The distillation under reduced pressure is desirably carried out at a low temperature in order to prevent the group VIII metal complex (I) from undergoing thermal degradation. Thus the distillation is desirably carried out at a temperature of 30 to 100°C and under a pressure of 10 to 300 mmHg. The water is desirably distilled off to such an extent that the obtained condensate containing the catalyst component permits, when it is re-used for the hydroformylation, no isolated water to be present in the reaction medium. The catalyst components thus obtained can be re-used for the hydroformylation.

The lithium sulfonate (I) used in the present invention, such as lithium p-diphenylphosphinobenzenesulfonate, can also be prepared by other processes than the above. For example, lithium diphenylphosphide is produced by reacting diphenylphosphine with an alkyl lithium such as butyl lithium, and then the obtained lithium diphenylphosphide is reacted with lithium p-chlorobenzenesulfonate to obtain the desired product, which is then purified by column purification or like methods.

### EXAMPLES

Hereinbelow, the present invention is described by reference to Examples, which are by no means limitative of the invention. In the Examples that follow, unless otherwise specified, phosphorus compounds were synthesized under an atmosphere of nitrogen or argon, and hydroformylation and water extraction were carried out under an atmosphere of a 1:1 by mole mixed gas of H₂/CO.

Chlorine, lithium, sodium and potassium were quantitatively determined by ion chromatography (with DX-120 made by Nippon Dionex K.K.). p-Chlorobenzenesulfonates were quantitatively determined with a ¹H-NMR spectrometer (Lambda-500, made by JEOL LTD.) and p-diarylphosphinobenzenesulfonates and oxides thereof with a ³¹P-NMR spectrometer (Lambda-500, made by JEOL LTD.). The product of hydroformylation of 7-oten-1-al was quantitatively determined by gas chromatography (with GC-14B, made by SHIMADZU CORPORATION).

### Reference Example 1

### Synthesis of potassium p-diphenylphosphinobenzenesulfonate

A 1-liter three-necked flask equipped with a reflux condenser, dropping funnel, thermometer and magnetic stirrer was charged with 700 ml of tetrahydrofuran and then 29 g (0.74 mole) of metallic potassium. The contents were refluxed for 0.5 hour, to give a dispersion of metallic potassium. To the dispersion, 83 g (0.376 mole) of chlorodiphenylphosphine was added dropwise over 1.2 hours and then the mixture was refluxed for 1 hour to give a solution of potassium diphenylphosphide. To the solution, the temperature of which had been set at 35°C, 74 g (0.373 mole) of lithium p-chlorobenzenesulfonate was added and the contents were stirred for 45 minutes at a bath temperature of 50°C. After completion of reaction, 350 ml of tetrahydrofuran was distilled off from the obtained reaction mixture. To the resultant solution 300 ml of diisopropyl ether and 700 ml of water were added, and extraction was effected to obtain a mixed layer comprising an aqueous layer and a tetrahydrofuran layer. The mixed layer was washed with 300 ml of diisopropyl ether and an aqueous layer was separated. The aqueous layer was filtered and then condensed to a volume 2/3 the original volume. The condensed liquid was ice-cooled down to 10°C and colorless solid that precipitated was taken by filtration. The colorless solid was recrystallized from water twice, to yield 73 g (yield: 51% based on chlorodiphenylphosphine) of potassium p-diphenylphosphinobenzenesulfonate having the following properties.

The cation was all potassium ion. The contents of chloride ion, the oxide of potassium p-diphenylphosphinobenzenesulfonate and potassium p-chlorobenzenesulfonate were 0.006 mole %, 0.47 mole % and not more than 0.03 mole %, respectively.
¹H-NMR (500 MHz, heavy water, TSP, ppm): δ =7.3 ppm (m, 14H), 7.7 ppm (d,2H)
³¹P-NMR (500 MHz, heavy water, phosphoric acid, ppm): δ =-5.37 ppm (s, P)

### Reference Example 2

### Synthesis of potassium p-diphenylphosphinobenzenesulfonate

A 1-liter three-necked flask equipped with a reflux condenser, dropping funnel, thermometer and magnetic stirrer was charged with 200 ml of dibutyl ether and then 20 g (0.87 mole) of metallic sodium. The contents were stirred for 0.5 hour at a liquid phase temperature of 100°C, to give a dispersion of metallic sodium. To the dispersion, 97 g (0.44 mole) of chlorodiphenylphosphine was added dropwise over 2 hours at such a rate as to maintain the liquid phase temperature at 100 to 110°C and then the mixture was stirred for 1 hour at the same temperature to give sodium diphenylphosphide. To the solution, the temperature of which had been set at 35°C, 250 ml of tetrahydrofuran was added. Separately, a 2-liter three-necked flask equipped with a reflux condenser, dropping funnel, thermometer and mechanical stirrer was charged with 89 g (1.20 mole) of potassium chloride, 110 g (0.55 mole) of lithium p-chlorobenzenesulfonate and 750 ml of tetrahydrofuran and the contents were stirred for 30 minutes under reflux. To the obtained mixture, the sodium diphenylphosphide prepared above was added through the dropping funnel over 2 hours at such a rate as to maintain the liquid phase temperature at 60 to 70°C, and the mixture was stirred for 1 hour at the same temperature to give a reaction mixture. To the reaction mixture, 1.5 l of saturated aqueous potassium chloride solution was added at room temperature to effect reaction and the resulting bottom layer was removed by separation. Two similar procedures were repeated each with 750 ml of saturated aqueous potassium chloride solution. To the organic layer obtained 300 ml of water was added and an aqueous layer was obtained by separation. The aqueous layer was subjected to distillation to remove tetrahydrofuran, then ice-cooled to 10°C, and colorless solid that precipitated was taken by filtration. The colorless solid was crystallized from water once, to yield 112 g (yield: 67% based on chlorodiphenylphosphine) of potassium p-diphenylphosphinobenzenesulfonate.

The cations consisted of 99% potassium ion and 1% sodium ion. The contents of chloride ion, the oxide of potassium p-diphenylphosphinobenzenesulfonate and potassium p-chlorobenzenesulfonate were 0.004 mole %, 0.12 mole % and not more than 0.03 mole %, respectively.

### Reference Example 3

### Synthesis of potassium p-diphenylphosphinobenzenesulfonate

A 1-liter three-necked flask equipped with a reflux condenser, dropping funnel, thermometer and magnetic stirrer was charged with 200 ml of dibutyl ether and then 20 g (0.87 mole) of metallic sodium. The contents were stirred for 0.5 hour at a liquid phase temperature of 100°C, to give a dispersion of metallic sodium. To the dispersion, 97 g (0.44 mole) of chlorodiphenylphosphine was added dropwise over 2 hours at such a rate as to maintain the liquid phase temperature at 100 to 110°C and then the mixture was stirred for 1 hour at the same temperature to give sodium diphenylphosphide. To the solution, the temperature of which had been set at 35°C, 250 ml of tetrahydrofuran was added. Separately, a 2-liter three-necked flask equipped with a reflux condenser, dropping funnel, thermometer and mechanical stirrer was charged with 110 g (0.55 mole) of lithium p-chlorobenzenesulfonate and 750 ml of tetrahydrofuran and the contents were stirred for 30 minutes under reflux. To the obtained mixture, the sodium diphenylphosphide prepared above was added through the dropping funnel over 2 hours at such a rate as to maintain the liquid phase temperature at 60 to 70°C, and the mixture was stirred for 1 hour at the same temperature to give a reaction mixture. To the reaction mixture, 1.5 1 of saturated aqueous potassium chloride solution was added at room temperature to effect reaction and the resulting bottom layer was removed by separation. Three similar procedures were repeated each with 750 ml of saturated aqueous potassium chloride solution. To the organic layer obtained 300 ml of water was added and an aqueous layer was obtained by separation. The aqueous layer was subjected to distillation to remove tetrahydrofuran, then ice-cooled to 10°C, and colorless solid that precipitated was taken by filtration. The colorless solid was recrystallized from water once, to yield 107 g (yield: 64% based on chlorodiphenylphosphine) of potassium p-diphenylphosphinobenzenesulfonate.

The cations consisted of 99% potassium ion and 1% sodium ion. The contents of chloride ion, the oxide of potassium p-diphenylphosphinobenzenesulfonate and potassium p-chlorobenzenesulfonate were 0.005 mole %, 0.12 mole % and not more than 0.03 mole %, respectively.

### Example 1

### Synthesis of lithium p-diphenylphosphinobenzenesulfonate

A 1-liter three-necked flask equipped with a reflux condenser, thermometer and magnetic stirrer was charged with 200 ml of water and 300 ml of methyl alcohol and, further, 50 g (130 mmoles) of the potassium p-diphenylphosphinobenzenesulfonate obtained in Reference Example 1 and 50 g (390 mmoles) of lithium sulfate monohydrate. The contents were refluxed for 2 hours. After the reaction mixture obtained had been allowed to cool to room temperature, 2 l of acetone was added and solid matter was removed by filtration. The filtrate was condensed and dried in a rotary evaporator, to give as a white solid 42 g (yield: 92%) of lithium p-diphenylphosphinobenzenesulfonate having the following properties.

The cation was all lithium ion. The contents of chloride ion, the oxide of lithium p-diphenylphosphinobenzenesulfonate and lithium p-chlorobenzenesulfonate were 0.0133 mole %, 0.32 mole % and not more than 0.03 mole %, respectively.
¹H-NMR (500 MHz, heavy water, TSP, ppm): δ =7.3 ppm (m, 14H), 7.7 ppm (d,2H)
³¹P-NMR (500 MHz, heavy water, phosphoric acid, ppm): δ =-5.39 ppm (s, P)

### Example 2

### Synthesis of lithium p-diphenylphosphinobenzenesulfonate

A 500-ml three-necked flask equipped with a reflux condenser, thermometer and magnetic stirrer was charged with 200 ml of water and, further, 50 g (130 mmoles) of the potassium p-diphenylphosphinobenzenesulfonate obtained in the same manner as in Reference Example 1 and 50 g (390 mmoles) of lithium sulfate monohydrate. The contents were refluxed for 2 hours. After the reaction mixture obtained had been allowed to cool to room temperature, 2 l of acetone was added and solid matter was removed by filtration. The filtrate was condensed and dried in a rotary evaporator, to give as a white solid 42 g (yield: 92%) of lithium p-diphenylphosphinobenzenesulfonate.

The cation was all lithium ion. The contents of chloride ion, the oxide of lithium p-diphenylphosphinobenzenesulfonate and lithium p-chlorobenzenesulfonate were 0.0133 mole %, 0.30 mole % and not more than 0.03 mole %, respectively.

### Example 3

### Synthesis of lithium p-diphenylphosphinobenzenesulfonate

A 500-ml three-necked flask equipped with a reflux condenser, thermometer and magnetic stirrer was charged with 200 ml of water and, further, 50 g (130 mmoles) of the potassium p-diphenylphosphinobenzenesulfonate obtained in the same manner as in Reference Example 1 and 50 g (390 mmoles) of lithium sulfate monohydrate. The contents were refluxed for 30 minutes. After the reaction mixture obtained had been allowed to cool to room temperature, 2 l of acetone was added and solid matter was separated by filtration. The filtrate was condensed and dried in a rotary evaporator, to give as a white solid 42 g (yield: 92%) of lithium p-diphenylphosphinobenzenesulfonate.

The cation was all lithium ion. The contents of chloride ion, the oxide of lithium p-diphenylphosphinobenzenesulfonate and lithium p-chlorobenzenesulfonate were 0.0133 mole %, 0.30 mole % and not more than 0.03 mole %, respectively.

### Example 4

### Synthesis of lithium p-diphenylphosphinobenzenesulfonate

A 500-ml three-necked flask equipped with a reflux condenser, thermometer and magnetic stirrer was charged with 200 ml of water and, further, 50 g (130 mmoles) of the potassium p-diphenylphosphinobenzenesulfonate obtained in the same manner as in Reference Example 2 and 50 g (390 mmoles) of lithium sulfate monohydrate. The contents were refluxed for 30 minutes. After the reaction mixture obtained had been allowed to cool to room temperature, 2 l of acetone was added and solid matter was removed by filtration. The filtrate was condensed and dried in a rotary evaporator, to give as a white solid 42 g (yield: 92%) of lithium p-diphenylphosphinobenzenesulfonate.

The cation was all lithium ion. The contents of chloride ion, the oxide of lithium p-diphenylphosphinobenzenesulfonate and lithium p-chlorobenzenesulfonate were 0.007 mole %, 0.34 mole % and not more than 0.03 mole %, respectively.

### Example 5

### Synthesis of lithium p-diphenylphosphinobenzenesulfonate

A 500-ml three-necked flask equipped with a reflux condenser, thermometer and magnetic stirrer was charged with 200 ml of water and, further, 50 g (130 mmoles) of the potassium p-diphenylphosphinobenzenesulfonate obtained in Reference Example 3 and 50 g (390 mmoles) of lithium sulfate monohydrate. The contents were refluxed for 30 minutes. After the reaction mixture obtained had been allowed to cool to room temperature, 2 l of acetone was added and solid matter was removed by filtration. The filtrate was condensed and dried in a rotary evaporator, to give as a white solid 42 g (yield: 92%) of lithium p-diphenylphosphinobenzenesulfonate.

The cation was all lithium ion. The contents of chloride ion, the oxide of lithium p-diphenylphosphinobenzenesulfonate and lithium p-chlorobenzenesulfonate were 0.008 mole %, 0.27 mole % and not more than 0.03 mole %, respectively.

### Reference Example 4

### Synthesis of sodium m-diphenylphosphinobenzenesulfonate

A 1-liter three-necked flask equipped with a dropping funnel, thermometer and magnetic stirrer was charged with 150 ml of concentrated sulfuric acid and, while care was taken to maintain the liquid temperature at not more than 30°C, 150 g (0.57 moles) of triphenylphosphine, to obtain a solution of triphenylphosphine in concentrated sulfuric acid. To the solution 285 ml of fuming sulfuric acid containing 25% by weight sulfur trioxide was added dropwise over 2 hours while care was taken to maintain the liquid temperature at not more than 30°C. After completion of the addition, the mixture was stirred for 14 hours at a liquid temperature of not more than 30°C. The solution thus obtained was diluted with 5 l of ice-cooled water over 2 hours so that the liquid temperature was maintained at not more than 10°C. The solution in diluted sulfuric acid thus obtained was, by addition of 4 l of methyl isobutyl ketone, subjected to extraction treatment, to give an organic layer. The organic layer was neutralized by addition of about 300 ml of a 5% by weight aqueous sodium hydroxide solution. After completion of the neutralization, the solution was found to be separated into two layers, from which an aqueous layer was taken out. The aqueous layer was then washed with 250 ml of methyl isobutyl ketone. The aqueous layer was condensed to about 200 ml, then ice-cooled to a temperature of 10°C, and colorless solid that precipitated was taken by filtration. The colorless solid was recrystallized from water twice, to give 35 g (yield: 17%) of sodium m-diphenylphosphinobenzenesulfonate.

The cation was 100% sodium ion. The contents of chloride ion and the oxide of sodium m-diphenylphosphinobenzenesulfonate were 0.002 mole %, and 0.5 mole %, respectively.

### Example 6

### Hydroformylation with use of lithium p-diphenylphosphinobenzenesulfonate-rhodium complex catalyst and recovery of catalyst components

The steps here were all carried out under an atmosphere of a CO/H₂ (molar ratio: 1/1) mixed gas. A 100-ml three-necked flask equipped with a Teflon (registered trademark) magnetic rotor was charged with 3.9 mg (0.015 mmole) of Rh(acac)(CO)₂ and 421 mg (1.2 mmoles) of the lithium p-diphenylphosphinobenzenesulfonate obtained in Example 1, and further with 6 ml of polyglyme (polyethylene glycol). The contents were stirred for 30 minutes at 50°C, to give a homogeneous catalyst solution. A 50-ml three-necked flask equipped with a Teflon (registered trademark) magnetic rotor was charged with 3 ml of the catalyst solution thus prepared and 27 ml (0.167 mole, purity: 93%) of 7-octen-1-al, to obtain a mixed liquid. The mixed liquid thus obtained was fed to a 100-ml autoclave equipped with a gas inlet and a sampling port. The total pressure was set at 3.0 MPa and the inside temperature was elevated to 85°C with stirring, and then reaction was effected for 7 hours, to obtain 20.6 g (0.132 mole, yield: 79%) of 1,9-nonanedial and 4.4 g (0.028 mole, yield: 17%) of 2-methyloctanal. The conversion of 7-octen-1-al was 96%, and the ratio between the formation of the linear aldehyde and that of the branched aldehyde was 4.65:1.

Thereafter, 30 ml of the reaction mixture after completion of reaction was extracted with 9 ml of water. The aqueous layer obtained was condensed and dried to solid, to give 189 mg (recovery ratio: 82%) of lithium p-diphenylphosphinobenzenesulfonate.

### Example 7

### Hydroformylation with use of lithium p-diphenylphosphinobenzenesulfonate-rhodium complex catalyst and recovery of catalyst components

A 100-ml three-necked flask equipped with a Teflon (registered trademark) magnetic rotor was charged with 3.9 mg (0.015 mmole) of Rh(acac)(CO)₂ and 421 mg (1.2 mmoles) of the lithium p-diphenylphosphinobenzenesulfonate obtained in Example 2, and further with 6 ml of polyethylene glycol dimethyl ether. The contents were stirred for 30 minutes at 50°C, to give a homogeneous catalyst solution. A 50-ml three-necked flask equipped with a Teflon (registered trademark) magnetic rotor was charged with 3 ml of the catalyst solution thus prepared and 27 ml (0.167 mole, purity: 93%) of 7-octen-1-al, to obtain a mixed liquid. The mixed liquid thus obtained was fed to a 100-ml autoclave equipped with a gas inlet and a sampling port. The total pressure was set at 3.0 MPa and the inside temperature was elevated to 85°C with stirring, and then reaction was effected for 7 hours, to obtain 20.6 g (0.132 mole, yield: 79%) of 1,9-nonanedial and 4.4 g (0.028 mole, yield: 17%) of 2-methyloctanal. The conversion of 7-octen-1-al was 96%, and the ratio between the formation of the linear aldehyde and that of the branched aldehyde was 4.65:1. The rhodium concentration in this Example was about 20 ppm.

Thereafter, the reaction mixture was fed under pressure into a 50-ml three-necked flask, the inside of which had been sufficiently replaced by the mixed hydrogen/carbon monoxide gas, while care was taken not to contact the mixture with air. To the mixture, 9 ml of water was added. The obtained mixture was stirred for 20 minutes with the inside temperature maintained at 30°C. After termination of the stirring, the bottom aqueous layer was withdrawn. Analysis by liquid chromatography reveals that the recovery ratio of lithium p-diphenylphosphinobenzenesulfonate was 82%. ICP emission spectrography shows that the recovery ratio of rhodium was 97%.

### Example 8

### Hydroformylation with use of lithium p-diphenylphosphinobenzenesulfonate-rhodium complex catalyst and recovery of catalyst components

Example 7 was repeated except that the amount of lithium p-diphenylphosphinobenzenesulfonate was changed from 421 mg (1.2 mmoles) to 105 mg (0.3 mmole), the reaction pressure to 1 MPa and the reaction time to 4 hours, to obtain 21.1 g (0.135 mole, yield: 81%) of 1,9-nonanedial and 4.1 g (0.026 mole, yield: 16%) of 2-methyloctanal. The conversion ratio of 7-octen-1-al was 97% and the ratio between the formation of the linear aldehyde and that of the branched aldehyde was 5.06:1.

Thereafter, the procedure of Example 7 was followed to separate an organic layer and an aqueous layer. Analysis by liquid chromatography reveals that the recovery ratio of lithium p-diphenylphosphinobenzenesulfonate was 83%. ICP emission spectrography shows that the recovery ratio of rhodium was 97%.

### Comparative Example 1

### Hydroformylation with use of potassium p-diphenylphosphinobenzenesulfonate-rhodium complex catalyst and recovery of catalyst components

Example 6 was repeated except that instead of 421 mg (1.2 mmoles) of lithium p-diphenylphosphinobenzenesulfonate 460 mg (1.2 mmoles) of the potassium p-diphenylphosphinobenzenesulfonate obtained in Reference Example 1 was used and that the reaction time was changed to 3 hours, to obtain 18.8 g (0.121 mole, yield: 72%) of 1,9-nonanedial and 6.0 g (0.039 mole, yield: 23%) of 2-methyloctanal. The conversion ratio of 7-octen-1-al was 95% and the ratio between the formation of the linear aldehyde and that of the branched aldehyde was 3.13:1.

Thereafter, 30 ml of the reaction mixture after completion of reaction was extracted with 9 ml of water. The aqueous layer obtained was condensed and dried to solid, to give 76 mg (recovery ratio: 33%) of potassium p-diphenylphosphinobenzenesulfonate.

### Comparative Example 2

### Hydroformylation with use of potassium p-diphenylphosphinobenzenesulfonate-rhodium complex catalyst and recovery of catalyst components

Example 7 was repeated except that instead of 421 mg (1.2 mmoles) of lithium p-diphenylphosphinobenzenesulfonate 460 mg (1.2 mmoles) of the potassium p-diphenylphosphinobenzenesulfonate obtained in Reference Example 1 was used and that the reaction time was changed to 3 hours, to obtain 18.8 g (0.121 mole, yield: 72%) of 1,9-nonanedial and 6.0 g (0.039 mole, yield: 23%) of 2-methyloctanal. The conversion ratio of 7-octen-1-al was 95% and the ratio between the formation of the linear aldehyde and that of the branched aldehyde was 3.13:1.

Thereafter, the procedure of Example 7 was followed to separate an organic layer and an aqueous layer. Analysis by liquid chromatography reveals that the recovery ratio of potassium p-diphenylphosphinobenzenesulfonate was 33%. ICP emission spectrography shows that the recovery ratio of rhodium was 40%.

### Comparative Example 3

### Hydroformylation with use of sodium m-diphenylphosphinobenzenesulfonate-rhodium complex catalyst and recovery of catalyst components

Example 7 was repeated except that instead of 421 mg (1.2 mmoles) of lithium p-diphenylphosphinobenzenesulfonate 440 mg (1.2 mmoles) of the sodium m-diphenylphosphinobenzenesulfonate obtained in Reference Example 4 was used and that the reaction time was changed to 8 hours, to obtain 20.1 g (0.129 mole, yield: 73%) of 1,9-nonanedial and 7.0 g (0.045 mole, yield: 27%) of 2-methyloctanal. The conversion ratio of 7-octen-1-al was 100% and the ratio between the formation of the linear aldehyde and that of the branched aldehyde was 2.70:1.

Thereafter, the procedure of Example 7 was followed to separate an organic layer and an aqueous layer. Analysis by liquid chromatography reveals that the recovery ratio of sodium m-diphenylphosphinobenzenesulfonate was 70%. ICP emission spectrography shows that the recovery ratio of rhodium was 94%.

As is apparent from Comparative Example 1 and Comparative Example 2, use of potassium p-diphenylphosphinobenzenesulfonate leads to a low ratio of the obtained linear aldehyde to the branched aldehyde of 3.13 and to low recovery ratios of the catalyst components. In contrast, use of the lithium p-diphenylphosphinobenzenesulfonate of the present invention leads to a ratio of the obtained linear aldehyde to the branched aldehyde of at least 4.65 and high recovery ratios of the catalyst component. On the other hand, as shown in Comparative Example 3, although use of sodium m-diphenylphosphinobenzenesulfonate leads to a high recovery ratio of the catalyst components, it leads to a low ratio of the obtained linear aldehyde to the branched aldehyde of 2.70. In summary, the process of the present invention is excellent both in the selectivity to linear aldehydes and in economy.

### INDUSTRIAL APPLICABILITY

According to the present invention, lithium p-diarylphosphinobenzenesulfonates with high purity can be commercially advantageously produced at high yields.

According to the present invention, there are provided group VIII metal complexes having excellent economy in hydroformylation of ethylenically unsaturated compounds and giving the desired linear aldehydes at high selectivity. Use of the group VIII metal complexes leads to easy and commercially advantageous production of aldehydes by hydroformylation. Further according to the present invention, the catalyst components used in the hydroformylation can be recovered from the reaction mixture easily at high yield.

## Claims

1. A lithium p-diarylphosphinobenzenesulfonate represented by the general formula (I) 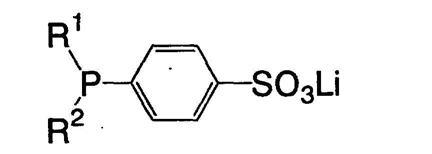 wherein R¹ and R² each represents an aryl group which may be substituted.

2. The lithium p-diarylphosphinobenzenesulfonate according to Claim 1, wherein said aryl groups represented by R¹ and R² are both phenyl group.

3. The lithium p-diarylphosphinobenzenesulfonate according to Claim 1, being lithium p-diphenylphosphinobenzenesulfonate.

4. A process for producing lithium p-diarylphosphinobenzenesulfonates represented by the general formula (I) 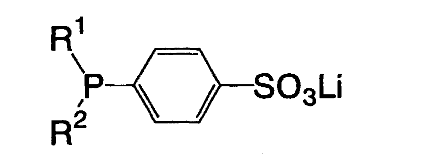 wherein R¹ and R² each represents an aryl group which may be substituted, which comprises reacting a potassium p-diarylphosphinobenzenesulfonate represented by the general formula (II) 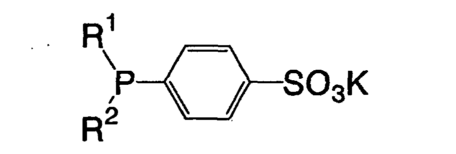 wherein R¹ and R² are as defined above, with the lithium salt of an acid having a solubility in water of 5 to 30% by weight, in the presence of water or a mixture of water and a water-miscible organic solvent.

5. The process according to Claim 4, wherein said aryl groups represented by R¹ and R² are both phenyl group.

6. The process according to Claim 4, wherein said potassium p-diarylphosphinobenzenesulfonate is potassium p-diphenylphosphinobenzenesulfonate.

7. The process according to Claim 4, wherein said lithium salt of an acid is carbonate, phosphate, phosphite, diphosphate, sulfate, sulfite or hydrochloride.

8. The process according to Claim 7, wherein said lithium salt of an acid is carbonate, sulfate or sulfite.

9. The process according to Claim 4, wherein said lithium salt of an acid is used in an amount ranging from 4 to 40 atoms of lithium atom contained in said lithium salt based on 1 atom of potassium contained in said potassium p-diarylphosphinobenzenesulfonate.

10. The process according to Claim 9, wherein said lithium salt of an acid is used in an amount ranging from 6 to 10 atoms of lithium atom contained in said lithium salt based on 1 atom of potassium contained in said potassium p-diarylphosphinobenzenesulfonate.

11. The process according to Claim 4, wherein said reaction is carried out in the presence of water.

12. The process according to Claim 4, wherein water is used in an amount of 1 to 50 parts by weight based on 1 part by weight of said potassium p-diarylphosphinobenzenesulfonate.

13. The process according to Claim 12, wherein water is used in an amount of 2 to 20 parts by weight based on 1 part by weight of said potassium p-diarylphosphinobenzenesulfonate.

14. A group VIII metal complex comprising a group VIII metal compound and, coordinating thereto, a lithium p-diarylphosphinobenzenesulfonate represented by the general formula (I) 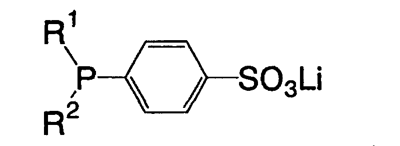 wherein R¹ and R² each represents an aryl group which may be substituted.

15. The group VIII metal complex according to Claim 14, wherein said aryl groups represented by R¹ and R² are both phenyl group.

16. The group VIII metal complex according to Claim 14, wherein said lithium p-diarylphosphinobenzenesulfonate is lithium p-diphenylphosphinobenzenesulfonate.

17. The group VIII metal complex according to Claim 14, wherein said group VIII metal compound is a rhodium compound, cobalt compound, ruthenium compound or iron compound having a catalytic activity for hydroformylation.

18. The group VIII metal complex according to Claim 17, wherein said group VIII metal compound is a member selected from the group consisting of RhO, Rh₂O, Rh₂O₃, RhO₂, rhodium nitrate, rhodium sulfate, rhodium chloride, rhodium iodide, rhodium acetate, Rh₄(CO)₁₂, Rh₆(CO)₁₆, RhCl(CO)(PPh₃)₂, RhCl(PPh₃)₃, RhBr(CO)(PPh₃)₂, RhCl(CO)(AsPPh₃)₂ and Rh(acac)(CO)₂.

19. The group VIII metal complex according to Claim 18, wherein said group VIII metal compound is Rh(acac)(CO)₂.

20. The group VIII metal complex according to Claim 14, wherein said lithium p-diarylphosphinobenzenesulfonate is used in an amount ranging from 1 to 10000 moles in terms of phosphorus atom based on 1 mole of said group VIII metal compound in terms of said group VIII metal atom.

21. The group VIII metal complex according to Claim 20, wherein said lithium p-diarylphosphinobenzenesulfonate is used in an amount ranging from 2 to 1000 moles in terms of phosphorus atom based on 1 mole of said group VIII metal compound in terms of said group VIII metal atom.

22. A process for producing aldehydes which comprises, on hydroformylating ethylenically unsaturated compounds with carbon monoxide and hydrogen in the presence of a catalyst to produce the corresponding aldehydes, using as the catalyst the group VIII metal complex according to Claim 14.

23. The process according to Claim 22, wherein said carbon monoxide and hydrogen are used in a molar H₂/CO ratio as inlet gas composition of 0.1 to 10.

24. The process according to Claim 23, wherein said molar H₂/CO ratio is 0.5 to 2.

25. The process according to Claim 22, wherein the reaction pressure is in a range of 0.1 to 10 MPa.

26. The process according to Claim 25, wherein said reaction pressure is in a range of 0.5 to 5 MPa.

27. The process according to Claim 22, wherein the reaction temperature is in a range of 40 to 150°C.

28. The process according to Claim 27, wherein the reaction temperature is in a range of 60 to 130°C.

29. The process according to Claim 22, wherein said group VIII metal complex is used in an amount per liter of the reaction liquid of 0.0001 to 1000 milligram-atom in terms of the group VIII metal atom.

30. The process according to Claim 29, wherein said amount per liter of the reaction liquid is in a range of 0.005 to 10 milligram-atom in terms of the group VIII metal atom.

31. A process for recovering catalyst components from the reaction mixture obtained by the process for producing aldehydes according to Claim 22, which comprises contacting the reaction mixture with water to extract the catalyst components into an aqueous layer and then removing the water from the aqueous layer.

32. The process according to Claim 31, wherein water is used in an amount ranging from 1 to 200% by volume based on the volume of the reaction mixture.

33. The process according to Claim 32, wherein water is used in an amount ranging from 5 to 50% by volume based on the volume of the reaction mixture.

34. The process according to Claim 31, wherein said extraction is carried out at a temperature ranging from 20 to 90°C.

35. The process according to Claim 31, wherein said extraction is carried out under an atmosphere of a mixed gas comprising hydrogen and carbon monoxide.

36. The process according to Claim 31, wherein the water is removed at a temperature of 30 to 100°C and under a pressure of 10 to 300 mmHg.
